Europäisches Patentamt

European Patent Office

Office européen des brevets

(19)

(11) Publication number: **0 088 620**
**A2**

# EUROPEAN PATENT APPLICATION

(21) Application number: **83301204.0**

(22) Date of filing: **07.03.83**

(51) Int. Cl.³: **G 01 N 29/00, A 61 B 10/00**

(30) Priority: **05.03.82 JP 34773/82**

(71) Applicant: **OLYMPUS OPTICAL CO., LTD.,
43-2, 2-chome, Hatagaya Shibuya-ku, Tokyo 151 (JP)**

(43) Date of publication of application: **14.09.83
Bulletin 83/37**

(72) Inventor: **Nakada, Akio, No. 226 Olympus Nishi Hachioji
Corporation, 5-16-2 Sandacho Hachioji-shi Tokyo (JP)**
Inventor: **Matsuo, Kazumasa, 3-1-7-304 Ochiai Tama-shi,
Tokyo (JP)**

(84) Designated Contracting States: **AT BE CH DE FR GB IT
LI NL SE**

(74) Representative: **Kennedy, Victor Kenneth et al, G.F.
REDFERN & CO. Marlborough Lodge 14 Farncombe
Road, Worthing West Sussex BN11 2BT (GB)**

(54) An ultrasonic probe for diagnostic examination of the interior of body cavities.

(57) An ultrasonic probe for diagnostic examination of body cavity interiors has a chamber (41) containing transmitting medium (46) put in to fill the space of an ultrasonic wave oscillator transducer or the like, coated with and having a jacket cover (32) on its tip, has the chamber filled to be with an ultrasonic wave transmitting medium (46) to improve the introduction of ultrasonic waves into a body cavity, and a sealable pathway to the exterior is provided so that bubbles in said transmitting medium (46) can be easily removed, and medium replenished or replaced.

If bubbles are contained in the transmitting medium (46), or if the characteristics of the medium deteriorate, the quality of the ultrasonic diagnosis image will be reduced. By providing a communicating path (65) between the housing chamber (41) and the exterior, sealed with a closure member (66) which can be opened and closed as necessary, bubble removal and or replenishment or replacement of the medium is facilitated.

-1-

## AN ULTRASONIC PROBE FOR DIAGNOSTIC
## EXAMINATION OF THE INTERIOR OF
## BODY CAVITIES

This invention relates to ultrasonic probes for diagnostic examination of the interior of body cavities.

The use of ultrasonic probes for diagnostic examination to obtain information within a body cavity has recently come into favour, especially when used together with an endoscope wherein an insertion sheath is inserted into the body cavity so as to be able to optically observe the interior of the body cavity or to operate thereon, using a forceps.

With this ultrasonic probe ultrasonic wave pulses projected into a body, for example, propagate to be reflected by any discontinuity of a boundary surface of any acoustic impedance represented by the product of the density of the medium and the sound velocity, and therefore the reflected ultrasonic wave pulses received will provide information on the reflection intensity, which can be utilised for a diagnosis.

As compared with an X-ray device, such ultrasonic wave diagnostic probes have many advantages, as information on living body tissue can be easily obtained without using any image-forming agent, the living body tissue will not be damaged by the incident waves, and the probe is easier to handle and less dangerous than X-ray or radio-active apparatus. With recent improvements in the quality and quantity of the information obtained due to progress in ultrasonic wave techniques, such probes are becoming increasingly popular for clinical diagnosis in the medical field.

As compared with diagnosis wherein ultrasonic wave pulses are transmitted and received at a body surface, the diagnostic examination of the interior of body cavities with ultrasonic waves, wherein ultrasonic wave pulses are transmitted and received at a position adjacent a living body organ within a body cavity, comparatively high frequency ultrasonic waves can be transmitted and received, despite large attenuation in propagation, and therefore there are many advantages, as information that is readily analysable and of high precision can be obtained, and the process will not be adversely influenced by any hypodermic fat layer or the like interposed between objects, and therefore this process will tend to be used more and more in the future. In general, such ultrasonic probes for introduction into a body cavity will be used with an ancillary or integral endoscope for optical observation.

In such ultrasonic probes for internal diagnostic examination, an ultrasonic wave oscillator or like transducer for transmitting and receiving ultrasonic waves is contained within a probe head that is provided with a jacket cover on the tip, to be introduced into a body cavity, and the interior of the probe head is filled with an ultrasonic wave transmitting medium. If bubbles appear in the transmitting medium, or the characteristics of the transmitting medium deteriorate, the image quality of the obtained ultrasonic wave fault image will be reduced, and therefore it becomes necessary to remove the bubbles, or replenishing or replacing the transmitting medium. However, in a conventional construction of ultrasonic probe the above mentioned operations have proved to be difficult.

One object of the present invention is to provide an ultrasonic probe of a construction such that transmitting medium can be easily replaced, wherein any bubbles in the medium can be easily removed, and yet the chamber containing the transmitting medium can be kept well sealed.

According to one exemplary embodiment of the invention, there is provided an ultrasonic probe for diagnostic examination of the interior of body cavities in which an ultrasonic transducer, for transmitting ultrasonic waves and detecting received echoes, is contained within a chamber formed at the tip of an elongate tubular member for insertion into a body cavity, the member being provided with a sealed jacket cover transparent to ultrasonic waves transmitted through said window, the chamber being filled with an ultrasonic wave transmitting medium and a rotatable driven shaft providing a scanning rotation of said transducer or of a deflecting surface between said transducer and said window, any reflected echoes being received by said transducer and converted back to electrical signals, and a communicating path formed in said chamber and leading to a sealable opening in the outer wall of said elongate tubular member and provided with closure means facilitating access to the interior of said chamber to permit cleansing or replenishment of said wave transmitting medium.

The invention will now be described with reference to the drawings, in which:-

Figure 1 is a longitudinal sectional view showing the tip of an insertion sheath of a known prior art probe;

Figure 2 is a perspective view of the known probe of Figure 1 showing the tip with the jacket cover;

Figure 3 is a schematic perspective view of one exemplary embodiment of an ultrasonic probe in accordance with the invention, and fitted with an optical endoscope sighting tube;

Figure 4 is a longitudinal section showing details of the structure of the ultrasonic probe head of the embodiment illustrated in Figure 3;

Figure 5 is a cross-section on line A-A' of Figure 4;

Figure 6 is a cross-section on line B-B' of Figure 4;

Figure 7 is a cross-section on line C-C' of Figure 4;

Figure 8 is a longitudinal section on line O-D of Figure 7, showing the periphery of a communicating path and sealed opening for removing or supplying transmitting medium; and

Figure 9 is a longitudinal section on line O-E of Figure 7, showing the periphery of the communicating path and sealed opening for removing or supplying grease.

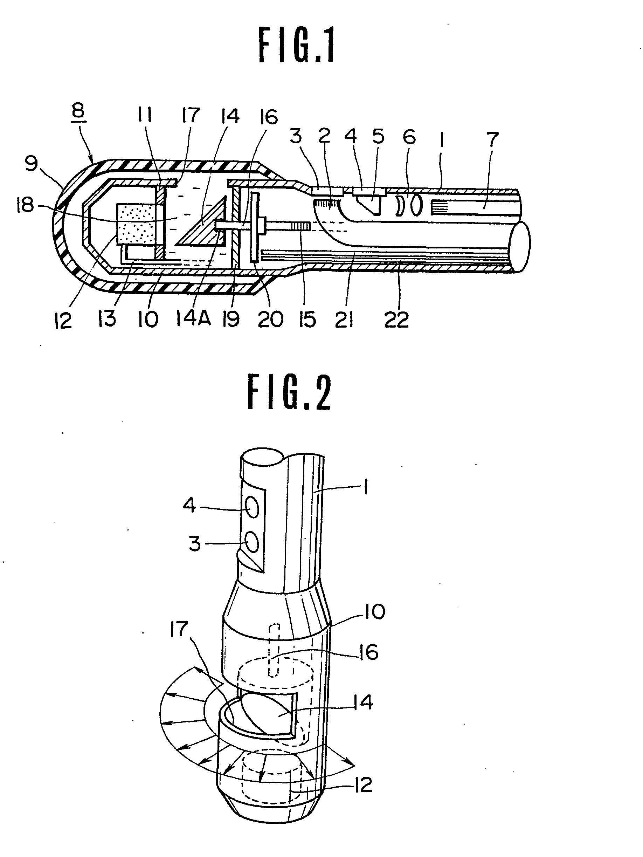

Before describing a preferred embodiment of the present invention, the known example of an ultrasonic probe for the diagnostic examination of the interior of body cavities will be discussed with reference to Figures 1 and 2.

A tubular insertion sheath 1 forming an ultrasonic wave probe contains an observing optical system, illuminating optical system, an ultrasonic scanning mirror on a rotating shaft and an electrical signal cable plus optical fibres for an ultrasonic wave oscillator.

A flexible light guide 2 formed of a glass fibre bunch or the like, feeds an illuminating light from an external light source (not illustrated), being inserted into the insertion sheath 1 and curved at its tip to project light through a glass window 3 into a body cavity.

An optical system for observing internal organs illuminated by the illuminating light is formed by a further glass window 4 adjacent to the glass window 3, a right angle prism 5 mounted so as to be in contact with the inside of this glass window 4, an image forming lens system 6 for forming an image of the incident light reflected at right angles through this prism 5 and an image guide 7 formed by a bundle of very fine glass fibres, transmitting the optical image formed by this image forming lens system 6 through this image guide 7 to an eyepiece so that the interior can be observed from outside.

A means for transmitting and receiving ultrasonic waves while rotating is contained in an ultrasonic wave probe head at the tip of the insertion sheath 1.

The probe head 8 has a soft jacket cover 9 formed of a rubber or an organic resin which can be used in close, direct contact with living body tissue, such as a stomach wall, and can effectively transmit and receive ultrasonic waves. An outer tube 10 formed of a metal or the like is contained within the jacket cover 9.

This outer tube 10 contains an ultrasonic wave oscillator transducer 12 mounted on a supporting member 11 fixed at its periphery to the outer tube 10, so as to generate and receive ultrasonic waves and convert them to electrical signals. A signal cable 13 is connected to this ultrasonic wave oscillator 12 to transmit electrical signals, an ultrasonic wave deflecting surface 14 transmits incident waves at right angles to the probe axis, and a shaft 16 rotates the ultrasonic wave deflecting surface 14, being driven via a flexible wire coil 15 extending from an external motor. In the back of the ultrasonic wave deflector 14 a screw hole 14A is formed, in which there is engaged a screw thread on the tip of the shaft 16, so that the ultrasonic wave deflector 14 can·be fixed to or detached from the shaft 16 by rotation.

The ultrasonic wave oscillator is formed of piezoelectric material, such as quartz or PZT (a solid solution of lead zirconate $PbZrO_3$ and lead titanate $PbTiO_3$), and serves to generate ultrasonic waves when driven by electrical signals, or generate electrical signals when excited by incident ultrasonic waves. The ultrasonic wave deflector 14 has a deflecting surface inclined at 45 degrees to deflect the ultrasonic waves substantially at right angles as they are transmitted from or return to the surface of this ultrasonic wave oscillator 12.

A window opening 17 is made in the outer tube 10 aligned with the rotary transmitting and receiving surface of the ultrasonic wave deflector 14 so that the beam of ultrasonic waves generated by the oscillator 12 may be projected (transmitted) over a wide scanning field, for example, about 180 degrees as indicated by arrows in Figure 2. An ultrasonic wave transmitting medium 18, such as a saline solution or olive oil, having substantially the same value of acoustic impedance as an organ within a living body, fills the space around the transmitting and receiving surface of the ultrasonic wave oscillator 12 and the ultrasonic wave deflector 14 so that ultrasonic waves may be effectively transmitted. This transmitting medium 18 fills the interior of the cover 9 through the window opening 17, but is sealed from the insertion sheath by a sealing member 19 so that the transmitting medium filling the ultrasonic wave chamber can not leak through to the observing optical system side. A disk 20 is fixed to the shaft 16 so as to rotate with the ultrasonic wave deflector 14, and is provided on one surface with a group of fine reflecting streaks. Two optical fibres 21 and 22 terminate opposite to this reflecting marking. One optical fibre 21 feeds light from an external light source to illuminate the surface of the disk 20. The other optical fibre 22 feeds light reflected by the reflective marking to an external control device (not illustrated) so as to signal the beginning of each sector scanning by ultrasonic waves.

This known construction of probe presents problems, in that the space within the insertion sheath 1 is so limited that if the ultrasonic wave oscillator 12 and ultrasonic wave deflector 14 were contained in an ultrasonic wave probe head 8 no larger in diameter than the insertion sheath 1, they would be so small that the intensity of the transmitted and received ultrasonic waves would be very weak and the signal/noise ratio of the indicated image would be reduced to an unacceptable level.

Therefore, the ultrasonic wave oscillator 12 and ultrasonic wave deflector 14, are made of sizes approaching the internal diameter of the enlarged outer tube 10, so that an adequate intensity of ultrasonic waves can be achieved.

When such an ultrasonic wave probe is used over a long period, gases dissolved in the transmitting medium 18 within the jacket cover 9 may form bubbles, especially if the sealing is not sufficiently robust, and bubbles may form during repair or maintenance, and these may deposit on the inner peripheral surface of the jacket cover 9, the inner and outer peripheral surfaces of the outer tube 10. Any bubbles adsorbed on the outer tube 10 or the like when gases are not vented well, or bubbles present in the transmitting medium 18 in the path of the ultrasonic waves whilst the transmitting medium 18 is agitated by the rotation of the ultrasonic wave deflector 14, and especially any bubbles deposited on the ultrasonic wave deflector 14, will all combine in reducing the image quality of the received ultrasonic wave fault image, to such a degree that they could lead to a faulty diagnosis.

Also, if any transmitting medium 18 leaks to the observing optical system side, the observed image will become unclear, and the transmitting medium will have to be supplemented. As the characteristics of the transmitting medium 18 deteriorate with age, the transmitting medium 18 will have to be replaced from time to time.

In the known ultrasonic wave probe illustrated the transmitting medium 18 can not be readily withdrawn and replaced, and bubbles are difficult to remove.

The present invention provides a construction which substantially avoids these problems of the above-described known example.

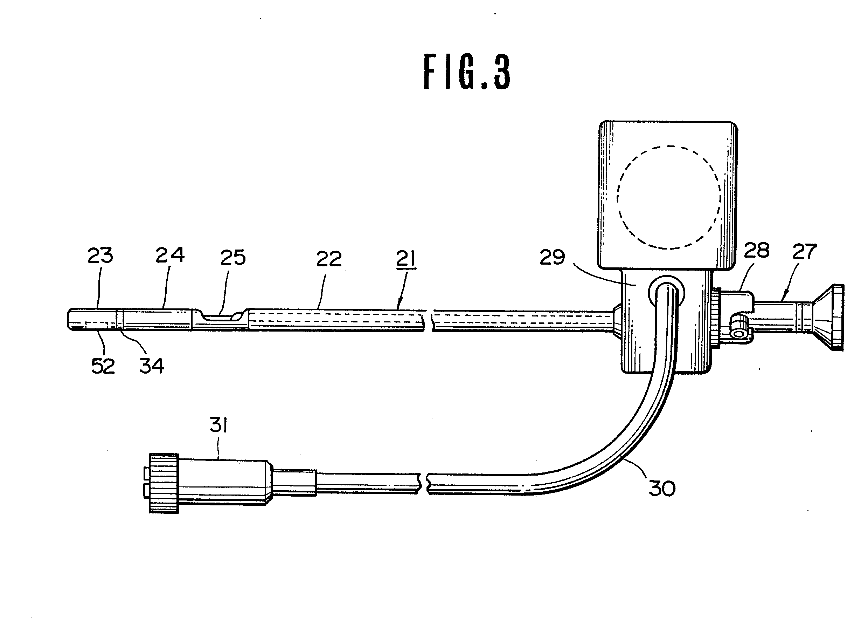

The exemplary embodiment of the present invention illustrated in Figures 3 to 9 will now be described.

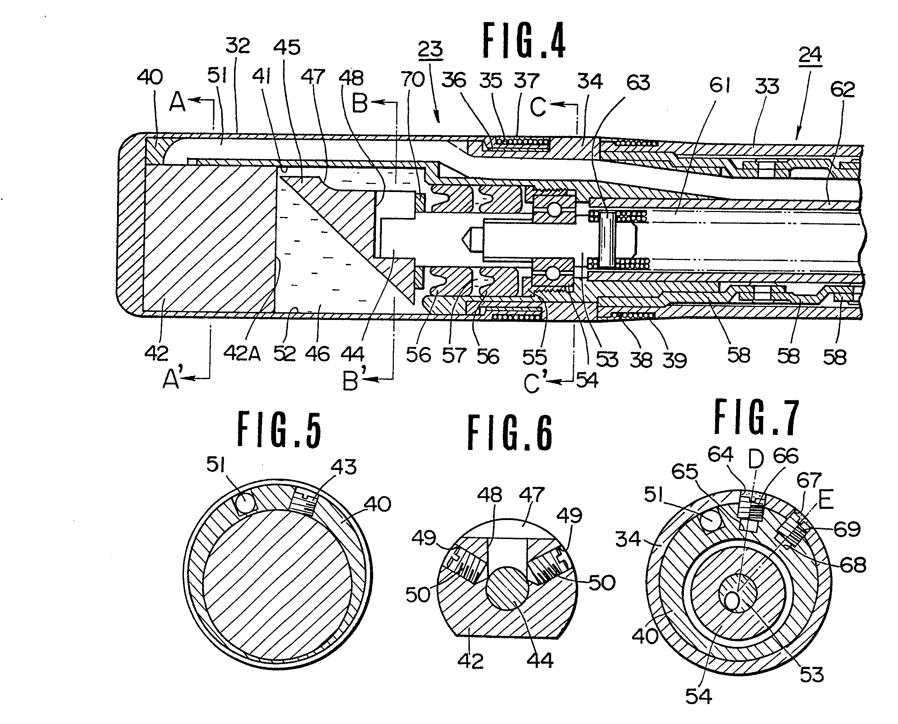

In these drawings the ultrasonic probe 21 shown in Figure 4, comprises at the tip portion an elongate insertion sheath 22 to be introduced into a body cavity, carrying a tubular ultrasonic wave probe head 23 for transmitting and receiving ultrasonic waves on a curvable part 24, an incised window 25 being provided in the outer periphery of the insertion sheath 22 to the rear of the curvable part 24, and a guide tube is provided for an optical sighting endoscope 27 to be inserted from the rear of the insertion sheath 22 to reach this incised window 25 and provide optical sighting.

An operating part 28 for inserting and removing the optical sighting tube 27 is provided at the rear end of the ultrasonic wave probe 21. A holding grip 29 is provided normal to the axial direction of the insertion sheath, and this contains a deflector driving motor, a low-noise head amplifier for amplifying the received echo signals, and a rotation detector for detecting the rotating position of a shaft rotated by the motor. An angle control knob for curving the above mentioned curvable part 24 is fitted to the side of this holding grip 29. A cable 30 transmitting electric power for the driving motor and the signal transmitting and receiving ultrasonic waves extends from the side of the grip opposite the side to which the angle control knob is fitted, and is provided at its tip with a liquid-tight connector 31, thus ensuring that the cable, the ultrasonic probe 21 and optical sighting tube 27 can be washed or disinfected.

The structure of the ultrasonic probe head 23 is shown in Figure 4.

The ultrasonic probe head 23 is covered at the tip with a soft, tubular jacket cover 32 closed at the tip but open at the rear to receive the probe head. This jacket cover is made of polyethylene or the like, and is kept sealed at the rear end by a thread-like member 35 wound in a recessed groove in a ring-shaped connecting fixing member 34 interposed on the outer periphery of a later described tip block near the boundary with a coating tube 33 on the outer periphery of the curvable part 24, and is fixed so securely that the cover will not come out of the groove.

That is to say, the jacket cover 32 is made smaller in diameter in the form of a step on the periphery of the rear end part, and is fitted in the smaller diameter groove in the recessed step of the connecting fixing member 34 fitted around it with a soft tube 36 of high elasticity and strongly fixed in the end part by the thread-like member 35 wound on the recessed step so as to improve the liquid-tight sealing. The outer periphery of this wound thread-like member 35 is painted with a bonding agent. A further recessed step is formed near the end of the soft coating tube 33 adjacent to the connecting fixing member 34. A thread-like member 38 is wound on this step and is painted with a bonding agent 39 in the outer peripheral recess. Thus, the soft jacket cover 32 on the tip side, and the soft coating tube 33 on the outer periphery of the curvable part 24 are not connected directly to each other, but have a ring-shaped connecting fixing member 34 of sufficient strength interposed between them so that the jacket cover 32 and coating tube 33 are strongly held, and able to withstand being repeatedly inserted and removed, should the jacket cover 32 on the tip side, or the coating tube 33 on the outer periphery of the curvable part 24 become worn or broken, only the one part need be replaced, by unwinding the above mentioned wound thread-like member 35 or 38, as the case may be.

A housing chamber 41 is formed in a hollow tip block 40 covered by the jacket. Within this housing chamber 41, an ultrasonic wave oscillator 42 transmitting and receiving ultrasonic waves is fixed to the above mentioned tip block 40 by a screw 43 or the like and a rotatable and removable ultrasonic wave deflector 45 is fitted to the tip of a first shaft 44 rotated and driven to scan ultrasonic waves, its surface being inclined, for example, by 45 degrees with respect to the ultrasonic wave transmitting and receiving surface 42A of the ultrasonic wave oscillator transducer 42.

The deflector 45 has an ultrasonic wave reflecting surface formed by a surface made by diagonally cutting a columnar member, and has an incised recess 47 (as shown in Figures 4 and 6) formed to the rear of this reflecting surface, so as to collect and remove bubbles produced within a wave transmitting medium 46, such as an aqueous solution of sucrose filling the housing chamber 41 around the mirror 45. If an aqueous solution of sucrose having a concentration of from 5 to 15% is used for the transmitting medium, the respective members will be less subject to corrosion than with saline solution or the like; the acoustic impedance can be made substantially equal to that of a living body, so that ultrasonic waves will be efficiently transmitted; the aqueous solution will have less loss components for the propagation of ultrasonic waves than in oil or the like, and therefore the intensity of ultrasonic wave beams will not be reduced during propagation.

A guide groove 48 is formed at the rear of the deflector 45 so that the deflector can be fitted to and removed from the rotating shaft 44, being inserted or removed in a direction at right angles to the shaft 44, and is removably fixed to the shaft 44 by opposed screws 50, screwed into respective screw holes 49.

A cable 51 transmitting the signals to produce transmitting and detect received ultrasonic waves is connected to the ultrasonic wave oscillator 42, passing through the insertion sheath 22 to the holding grip 29, and being finally connected to an external indicating device through the cable 30 from the side of the holding grip 29.

A window 52 for transmitting ultrasonic waves and receiving reflected echo signals is formed by an opening in the wall of the housing chamber 41 formed in the elongate tip member 40, around the deflector 45 so that the ultrasonic beam from the transmitting surface 42A of the ultrasonic wave oscillator transducer 42 will be incident on the deflector 45 and will be emitted out of the window opening 52 in a direction normal to the probe axis, passing through the jacket cover 32. Any reflected ultrasonic waves returning as echoes from the discontinuities in the boundary surfaces of the surroundings, which form an external acoustic imepdance, will be redirected by the deflector 45 to be incident upon the ultrasonic wave oscillator transducer 42.

The deflector 45 can be readily removed, using the guide groove 48, and taken out of the window opening 52 for maintenance or repair.

The deflector 45 is fitted to the tip of the first shaft 44, that is screwed onto a second shaft 53 rotatably borne by a bearing 54. The rear of this bearing 54 contacts a locking part projecting inside the elongate tip member 40 and a mating projection formed on the second shaft 53, and is supported at the other end by a ring-shaped lock screw 55, so as to be restrained from forward and rearward movement.

Annular sealing members 56 having a substantially U-shaped cross-section are fitted in an annular air gap between the outer peripheral surface of the first shaft 44 and the inner wall surface of the elongate tip member 40. These respective sealing members 56 have elastic members (not illustrated) fitted in the U-shaped recesses so as to be biased radially inward and outward at both ends of the U-shaped arms, so that the ends firmly contact the inner and outer peripheral surfaces. A grease-filled chamber 57 is formed between the recess of one U-shaped member 56 and the rear of the other sealing member 56, and is charged with a lubricant grease such as Mori-coat Grease, to prevent the transmitting medium 46 from leaking through to the bearing 54 and to positively limit frictional resistance when the shaft 44 in contact with the sealing members 56 is driven, so that the shaft 44 can rotate smoothly.

A sequence of articulated frames 58 having diametrically opposed pivoted parts rotatably connecting adjacent frames are contained within the curvable part 24 which is covered by the coating tube 33, so that the curvable part 24 can be curved out of the plane of the drawing in Figure 4 in either direction by drawing and relaxing an operating wire (not illustrated).

The articulated frame 58 at the tip end of the curvable part 24 is fixed to the outer peripheral surface at the rear end of the elongate tip member 40. A flexible drive shaft 61 connected at the front end to the second shaft 53, and at the other end to a rigid drive shaft (not illustrated) is inserted through a flexible tube 62 inside the hollow assembly of the articulated frames 58, which extends from the fixed part to the incised window 25 shown in Figure 3.

The flexible shaft 61 is formed of inner and outer wound layer coils. These coils are oppositely wound so that the outside diameter will not vary with the direction of rotation, and rotational drive will be effectively transmitted, even when curved. The coil turns of this flexible shaft 61 are consolidated at the tip end by soldering or silver brazing, so as to be easy to perforate, diametrically opposed holes being formed at the end by a drill or the like, and fitted to the rear end of the second shaft 53 until it contacts the projection, and is secured to the second shaft by a pin 63 passed and fixed through the opposed holes and a mating hole in the second shaft 53, so that rotation of the flexible drive shaft 61 is transmitted to the second shaft 53. This flexible drive shaft 61 is covered on its outer periphery with a soft tube 62 moulded of Teflon or the like, to reduce any friction when its inner peripheral surface contacts the rotating flexible drive shaft 61, and to endure repeated curving.

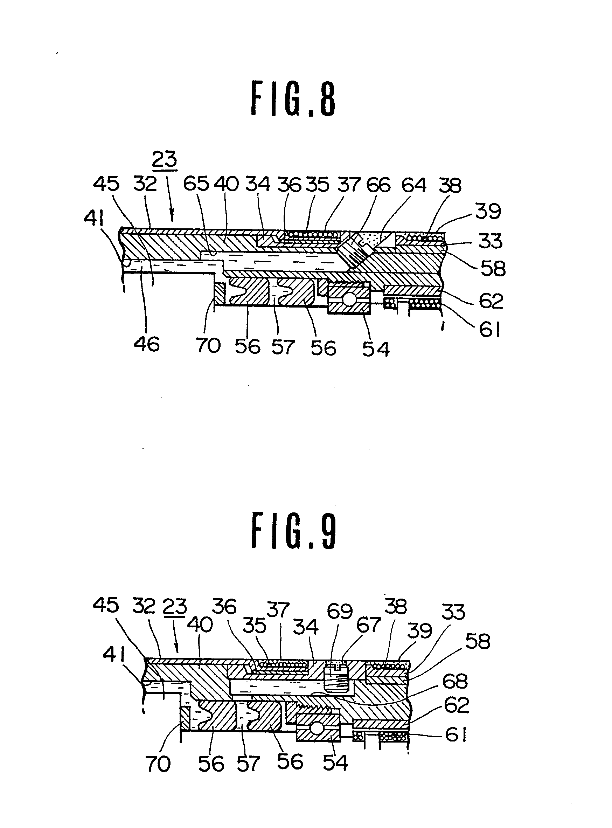

A screw-threaded hole 64 is formed, extending diagonally forward and inward, as shown in Figure 8, from a part of the outer peripheral surface of the ring-shaped connecting fixing member 34 toward the elongate tip member 40 inside it. A hole 65 forms a communicating pathway to the housing chamber containing the deflector 45 and filled with the transmitting medium 46, from the screw hole 64. A sealing plug 66 is screwed into the hole 64 so that, when this plug 66 is removed, a smaller diameter tube can be inserted into the screw hole 64 to easily remove bubbles produced within the transmitting medium 46 and to easily replenish or replace the transmitting medium 46.

As shown in Figures 7 and 9, a further screw-threaded hole 67 is directed toward the inside of the elongate member 40 in a position different from that of the hole 64 on the outer peripheral surface of the connecting fixing member 34, and extended forwardly and then inwardly to form a communicating pathway 68 to the grease charged chamber 57. A sealing plug 69 is screwed into the hole 67, and when this plug 69 is removed grease can readily be removed or forced in. An annular member 70 fitted to the shaft 44 adjacent the rear end of the deflector 45 is to prevent the sealing member 56 from moving forward, and can be omitted if the rear face of the deflector 45 is extended rearwardly.

The operation of the thus formed embodiment will now be described.

As shown in Figure 3, the optical sighting endoscope tube 27 is inserted into the guide tube of the ultrasonic probe 21 so that a view forward and to the right can be obtained through the incised window 25 via the observing optical system arranged in the tip part of the optical sighting endoscope tube 27. The cable 30 extending out of the holding grip 29 is connected to the observation indicating device through the separate unit containing the pulse oscillator, or the like, or directly. The light guide cable is connected to the optical sighting tube 27 so that illuminating light can be fed from the light source.

In the above mentioned state, the ultrasonic probe 21 is fitted to a trocar and introduced into a body cavity to permit observation of an object via the optical sighting endoscope tube 27, and the window opening 52 is pressed against the object such as an internal organ, by operation of the curvable part 24 if necessary. If the part 24 is curved to the right, the ultrasonic probe 23 and window opening 52, together with the object to be examined will be brought into the centre of the visual field.

With the window opening pressed against the object that is to be examined, when the motor within the holding grip 29 is energised, torque will be transmitted to the rigid shaft inserted in the ultrasonic probe 21 via the flexible shaft, not illustrated, the rotation of this rigid shaft will be transmitted to the flexible shaft 61 near the incised window 25, the rotation of this flexible shaft 61 will be transmitted onto the second and first shafts 53 and 44, and the deflector 45 fitted to the tip of the first shaft 44 will be driven to rotate.

When the deflector 45 is rotated, as pulses exciting ultrasonic waves are fed in sequence to the ultrasonic transducer 42 through the cable 51, a beam of ultrasonic waves will be emitted from the surface 42A for each pulse, and this beam will strike the surface of the deflector 45 after passage through the transmitting medium 46 and will be directed radially to be emitted to the object in contact with the outer peripheral surface at the window opening 52 after passage through the intervening transmitting medium 46. The ultrasonic beam emitted to the object will propagate on and gradually reduce in intensity due to absorption, but components will be reflected by any discontinuity of a boundary surface constituting an acoustic impedance, and become an ultrasonic echo signal, which returns to be re-directed by the deflector 45 and be incident upon the ultrasonic transducer 42. The ultrasonic echo signal returning to the ultrasonic transducer 42 will be converted to an electrical signal by the ultrasonic transducer, and will be amplified by the head amplifier (not illustrated) within the holding grip 29 after passage through the cable 51. This amplification avoids any reduction in the signal/noise ratio, and the amplified signal will be transmitted to the observation indicating device, to modulate the brightness of an oscilloscope beam, for example, provided as an indicating device sweep signal synchronised with the rotating position of the deflector 45 detected by the rotation detector (not illustrated) provided within the holding grip 29, or will be converted so that the amplitude of the reflection will vary the hue and will be indicated as an ultrasonic wave fault image.

In the embodiment formed to thus operate, as the elongate tip member 40 corresponding to the outer tube 10 in the above described conventional example is covered by the snuggly fitted jacket cover 32, there is no space at the outer peripheral surface of the elongate tip member which bubbles can enter, and be hard to remove. By provision of the communicating pathway 65 through the elongate tip member 40 from a sealable opening in the outer periphery of the connecting fixing member 34 at the rear of the ultrasonic probe 23, communicating with the housing chamber 41 that is charged with the transmitting medium 46, if this communicating pathway 65 is positioned to be on the upper side when the sealing plug 66 is unscrewed, bubbles within the housing chamber 41 can be easily removed via the communicating pathway. If there has been a reduction in the amount of transmitting medium 46 within the housing chamber 41, it can be easily replenished via an inserted tube of smaller diameter, or the like.

If the characteristics of the transmitting medium 46 have deteriorated significantly due to variation with age, the transmitting medium 46 can be discharged by inserting the smaller diameter tube or the like through the communicating pathway 65 and replaced by a new filling of transmitting medium 46.

By forming the incised recess 47 for collecting bubbles on the deflector 45, if the incised recess 47 is made to be normally in the upper position when the mirror 45 is not being rotated and driven, bubbles produced within the housing chamber 41 will tend to collect in the incised recess 47, the number of bubbles deposited on the mirror surface will be reduced, and the collected bubbles can easily be removed through the communicating pathway 65.

By fitting the deflector 45 to the driven shaft 44 via the guide groove 48, it is removable in a direction at right angles to the axial direction of the shaft 44, so that when the jacket cover 32 is removed the deflector 45 can be easily removed and re-fitted via the window opening, and repair and maintenance will be facilitated.

Annular sealing members 56 that are substantially U-shaped in cross-section and fitted one behind the other in the air gap between the outer periphery of the shaft 44 and the inner peripheral surface of the elongate tip member 40 surrounding the shaft 44 forms a chamber 57 between the sealing members 56 which can be charged with grease so that the liquid-tightness may be improved to prevent the transmitting medium 46 from leaking toward the bearing 54, and the shaft 44 may be rotated and driven without becoming eccentric. By providing the communicating path between the grease charged chamber 57 and the exterior sealed with the closure member screw 69 or the like to open and close the pathway, if the grease deteriorates in the characteristics due to the use over a long period, the grease can be easily replaced.

As the jacket cover 32 is made small in the outside diameter, the probe will be more easy to insert and remove than the prior art shown in Figure 1, and the pain given to the patient will be reduced. As the jacket cover 32 is strongly fixed at the open rear end by the thread-like member 35 holding it in the recess in the annular connecting fixing member 34 formed on the outer periphery of the probe when inserting or removing the probe the jacket cover 32 will be prevented from being separated. As the jacket cover 32 on the tip side and the coating tube 33 on the rear side are separately fixed to the connecting fixing member 34, even if the outer periphery of the probe is worn due to repeatedly being inserted and removed, or is damaged by repeated sterilisation or disinfection during use over a long period, it is possible to remove and replace only that part that is damaged by unwinding the thread-like member 35 or 38, thus giving added economy. As the outer periphery of the probe is durable, and can withstand sterilisation by disinfectants and as a connecting fixing member having mechanical strength is used, the outer periphery of the probe will be hard to damage and an extended working life can be anticipated.

As the rotation detector for detecting the position of the rotating deflector 45 is contained within the holding grip 29, the structure of the interior of the ultrasonic probe 23 is simplified, relative to the prior art, and even if the transmitting medium 46 or the grease does leak to the rear, the rotation detecting function will not be damaged. As the observation can be made from the fitted optical sighting endoscope tube via the incised window 25 to the rear of the curvable part 24, the ultrasonic probe 23 can be easily set in an objective position within the visual field.

As the flexible shaft 61 inserted through the curvable part 24 to transmit the drive torque is formed of oppositely wound coils in two layers, in whatever direction it is curved, each coil will compensate the other coil to smoothly transmit the torque.

The incised recess 47 is formed in the deflector 45 in the embodiment illustrated, but it can be provided in the elongate tip member 40 on the wall surface within the housing chamber 41 adjacent to the communicating pathway 65.

The communicating pathway 65 in the illustrated embodiment can be opened and closed by the sealing plug 66 screwed into the screw-threaded hole 64. However, for example, a cylindrical hole may be used instead of a screw-threaded hole, and the communicating pathway 65 may be closed by a piston fitting in this cylindrical hole and normally biased inwardly. In such a case, for example, a resilient member such as a spring can be interposed at the rear of the piston and secured by a screw, so that the communicating pathway 65 can be opened and closed. With such a formation, even if the volume of the transmitting medium 46 within the housing chamber 41 and the volumes of the housing chamber 41 and the respective members within the housing chamber 41 vary with the temperature of the object when in use, or with the temperature applied during disinfection or washing, as the piston moves along the communicating pathway 65, any adverse influence of the volume variation, such as that an excessive increase or reduction in the pressure of the transmitting medium 46 to an extent such that effective sealing can not be maintained will be avoided.

In the illustrated embodiment, an optical sighting endoscope tube is a removable item, but it can be made an integral part in like manner.

In the illustrated embodiment, the deflector 45 is fitted to the rotatable shaft 44 opposite the fixed ultrasonic wave oscillator transducer 42, to transmit and receive ultrasonic waves in a sector, but the ultrasonic wave oscillator transducer can be fitted directly to the rotary shaft 44, and thus avoid the need for a deflector 45. In such a case, signals can be transmitted to and from the ultrasonic wave oscillator transducer by a brush contact that engages a contact fitted to the rotary shaft.

CLAIMS:-

1.     An ultrasonic probe for diagnostic examination
of the interior of body cavities in which an ultrasonic
transducer, for transmitting ultrasonic waves and
detecting received echoes, is contained within a chamber
formed at the tip of an elongate tubular member for
insertion into a body cavity, the member being provided
with a sealed jacket cover transparent to ultrasonic
waves transmitted through said window, the chamber being
filled with an ultrasonic wave transmitting medium and
a rotatable driven shaft providing a scanning rotation
of said transducer or of a deflecting surface between
said transducer and said window, any reflected echoes
being received by said transducer and converted back
to electrical signals, and a communicating path formed
in said chamber and leading to a sealable opening in
the outer wall of said elongate tubular member and
provided with closure means facilitating access to the
interior of said chamber to permit cleansing or
replenishment of said wave transmitting medium.

2.     An ultrasonic probe as claimed in Claim 1,
in which said closure means is a plug having an
external screw-thread to engage in a screw hole provided
at the end of the communicating path.

3.     An ultrasonic probe as claimed in Claim 1,
in which two annular sealing members that are each
substantially U-shaped in cross-section are fitted
about said rotatable driven shaft to form a grease
charged lubricating chamber between the two sealing
members.

4.     An ultrasonic probe as claimed in Claim 3,
in which said grease charged lubricating chamber is accessible through a communicating path leading to a sealable opening in the outer wall of said elongate tubular member and provided with closure means facilitating access to the interior of said lubricating chamber to permit replenishment of said grease.

5.     An ultrasonic probe as claimed in Claim 1, in which said jacket cover is fitted to extend back from the tip of the probe to a point adjacent said sealable opening but not covering said opening, said jacket cover being a snugly fitting soft tube secured at its rear end in an annular groove around the outer wall of said elongate tubular member, in which groove it is secured firmly by a thread-like member wound on the outer periphery.

# FIG.1

# FIG.2

# FIG.3

# FIG.4

# FIG.5

# FIG.6

# FIG.7

3/4

C0088620

# FIG.8

# FIG.9